# EUROPÄISCHE PATENTSCHRIFT

(11) **EP 0 759 295 B1**
(45) Veröffentlichungstag und Bekanntmachung des Hinweises auf die Patenterteilung: **04.03.1998**
(21) Anmeldenummer: 96112325.4
(22) Anmeldetag: 31.07.1996
(51) Int. Cl.: A61K 31/13

(54) **Verwendung von Adamantan-Derivaten zur Behandlung von Tinnitus**
Use of adamantan derivatives for the treatment of tinnitus
Utilisation de dérivés adamantane pour le traitement du tintement d'oreille

(30) Priorität: 02.08.1995 DE 19528388
(43) Veröffentlichungstag der Anmeldung: 26.02.1997
(73) Patentinhaber: Zenner, Hans Peter, Prof. Dr. med., 72070 Tübingen (DE)
(72) Erfinder: Zenner, Hans Peter, Prof. Dr. med., 72070 Tübingen (DE); Ruppersberg, J. Peter, Prof. Dr., 72072 Tübingen (DE); Busch, Andreas, Dr., 72074 Tübingen (DE)
(74) Vertreter: Patentanwälte Ruff, Beier, Schöndorf und Mütschele

(56) Entgegenhaltungen:
- EP-A- 0 392 059
- ACTA OTOLARYNGOL., Bd. 115, Nr. 2, März 1995, (STOCKH.), Seiten 236-240, XP000609443 EHRENBERGER K. ET AL.: "Receptor Pharmacological Models for Inner Ear Therapies with Emphasis on Glutamate Receptors: A Survey"
- OTORHINOLARYNGOL. NOVA, Bd. 5, Nr. 3-4, Mai 1995 - August 1995, Seiten 148-152, XP000609451 EHRENBERGER K. ET AL.: "Rezeptorpharmakologische Modelle für eine kausale Tinnitustherapie"

## Beschreibung

Die Erfindung betrifft eine Verwendung von Adamantan-Derivaten der Formel 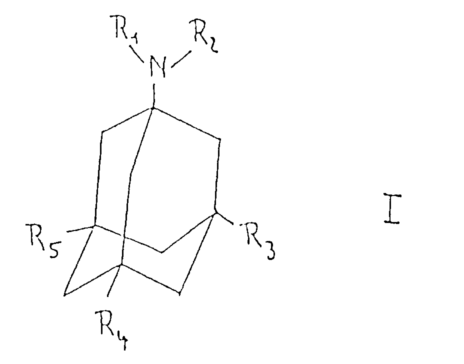 worin R₁ und R₂
- gleich oder verschieden sind und Wasserstoff oder geradkettige oder verzweigte Alkylgruppen mit 1 bis 6 C-Atomen sein können, oder
- zusammen mit dem N-Atom eine heterocyclische Gruppe mit 5 oder 6 Ringatomen darstellen können,
worin R₃ und R₄ gleich oder verschieden sind und Wasserstoff, geradkettige oder verzweigte Alkylgruppen mit 1 bis 6 C-Atomen, Cycloalkylgruppen mit 5 oder 6 C-Atomen oder die Phenylgruppe sein können,
und worin R₅ Wasserstoff oder eine geradkettige oder verzweigte Alkylgruppe mit 1 bis 6 C-Atomen sein können.

Die Verwendung von Adamantan-Derivaten, die unter die genannte allgemeine Formel I fallen, zur Therapie bestimmter Erkrankungen ist bereits bekannt. So wird beispielsweise der dopaminerge Einfluß von Amantadin (1-Adamantanamin) in einer Reihe von Veröffentlichungen beschrieben. Auch eine antivirale Wirkung bestimmter Amino-Adamantane ist nachgewiesen. Darüber hinaus zeigt die EP-B1-392059 die Verwendung von Adamantan-Derivaten zur Prävention und Behandlung der cerebralen Ischämie. Gemäß dieser Druckschrift kann durch die Adamantan-Derivate mit der oben beschriebenen Formel I die Zerstörung von Hirnzellen nach einer Ischämie protektiv verhindert werden. Dabei werden die Adamantan-Derivate als Antagonisten für die NMDA-Rezeptorkanäle der Nervenzellen im Gehirn eingesetzt. In der EP-B1-392059 sind sowohl allgemeine als auch spezielle Verfahren zur Herstellung der unter die oben genannte Formel I fallenden Adamantan-Derivate beschrieben.

Die Veröffentlichung von Ehrenberger und Felix in Acta Otalarygnol. (Stockholm) 1995; 115: 236-240 beschäftigt sich mit der Neurotransmission zwischen der inneren Haarzelle des Innenohrs und dem afferenten Neuron. Es wird versucht, gewisse Erkrankungen des Innenohrs in einem Modell durch eine sogenannte Glutamat-Neurotoxizität zu erklären. In diesem Zusammenhang wird angesprochen, Antagonisten zu verwenden, die die Glutamat-Bindungsstelle der postsynaptischen Membran blockieren. Hier wird es auch als denkbar bezeichnet, Antagonisten für die Glycin-Bindungsstelle, die redoxmodulierende Stelle oder für den NMDA-Rezeptorkomplex, zum Beispiel ein Adamantan-Derivat, zu verwenden. Das in der Veröffentlichung von Ehrenberger und Felix getestete Quinoxalin-Derivat Caroverin liefert allerdings bei der Behandlung von mutmaßlich glutamatinduziertem Tinnitus keine befriedigenden Ergebnisse.

Gleichzeitig muß man sich vor Augen halten, daß Erkrankungen des Innenohrs weit verbreitet sind. Dies gilt insbesondere für solche Erkrankungen oder Störungen, bei denen Ohrgeräusche, der sogenannte Tinnitus auftritt. Es wird geschätzt, daß in der Bundesrepublik Deutschland ca. 6 Millionen Menschen an Tinnitus leiden. Bei ca. 800000 Menschen ist der Tinnitus derart ausgeprägt, daß die Patienten intensiver ärztlicher Hilfe bedürfen. Der Patient ist durch quälende Ohrgeräusche massiv beeinträchtigt. Eine zuverlässige Therapie steht derzeit nicht zur Verfügung.

Im Hinblick auf die Komplexität von Innenohrerkrankungen, insbesondere der Tinnitusentstehung, die nicht abschließend aufgeklärt ist, wurden bisher eine Reihe von medikamentösen Therapien vorgeschlagen. Diese umfassen neben dem Einsatz von Anästhetika beispielsweise die Anwendung von Antiarrhythmika vom Lidocain-Typ oder von Antikonvulsiva, z. B. Carbamazepin. Meist führen derartige Behandlungen jedoch nicht zu überzeugenden Ergebnissen. Eine stationär durchgeführte Infusionstherapie mit Procain scheint die einzige Tinnitus-Therapieform, die einem Placebo, wenn auch nur gering, überlegen ist. Auch hier ist die Wirkung jedoch nur vorübergehend.

Die Erfindung stellt sich deshalb die Aufgabe, eine Behandlungsmöglichkeit für den Tinnitus zu schaffen, mit der eine spürbare Kompensation der mit der Erkrankung verbundenen Symptome erreicht wird. Insbesondere sollen die quälenden Ohrgeräusche zum Verschwinden gebracht oder doch zumindest so weit zurückgedrängt werden, daß der Patient seine normale Lebensqualität wiedererlangt.

Diese Aufgabe wird gelöst durch die beanspruchte Verwendung der Adamantan-Derivate der Formel I. Überraschend wurde festgestellt, daß der Tinnitus, der mit einem sogenannten positiven Recruitment und/oder einer Reduktion oder einem Ausfall der otoakustischen Emissionen verbunden ist, durch die Verwendung der genannten Verbindungen wirksam kompensiert werden kann.

Die Erfindung ist in Anspruch 1 dargestellt. Bevorzugte Ausführungsformen ergeben sich aus den Ansprüchen 2 bis 10. Der Wortlaut sämtlicher Ansprüche wird durch Bezugnahme zum Inhalt der Beschreibung gemacht.

Bei dem sogenannten positiven Recruitment handelt es sich um eine Erscheinung in der Audiometrie, wenn ein Lautstärkevergleich bei einseitiger Schwerhörigkeit vorgenommen wird. Bei positivem Recruitment benötigt man auf dem schwerhörigen Ohr eine geringere Verstärkung, um die gleiche Lautheitsempfindung wie auf dem gesunden Ohr hervorzurufen, d. h. der Hörverlust gleicht sich mit zunehmender Lautstärke aus. Bei den sogenannten otoakustischen Emissionen handelt es sich um im äußeren Gehörgang auftretende Schallereignisse, die mit dem Zustand des Mittel- oder Innenohrs im Zusammenhang stehen. Die otoakustischen Emissionen können spontan, d. h. ohne Reizung des Ohrs von außen, entstehen oder auch von außen evoziert werden, beispielsweise mit Hilfe eines Schallsenders.

Sowohl das Auftreten eines positiven Recruitments als auch die (Amplituden-)Reduktion oder der Ausfall der otoakustischen Emissionen lassen sich messen und somit können die betreffenden Patienten einer Innenohrfunktionsstörung zugeordnet werden.

Tritt bei einem Patienten ein Tinnitus auf, der mit einem positiven Recruitment und/oder Reduktion bzw. Verlust der otoakustischen Emissionen verbunden ist, so läßt dies den Schluß zu, daß eine Beeinträchtigung oder Funktionsstörung der äußeren Haarzellen mit ihrem cochleären Verstärker (cochleären Amplifier) vorliegt. Da diese Funktionsstörung bei der erfindungsgemäßen Verwendung gebessert oder aufgehoben wird, muß ein neuer Wirkmechanismus zugrundeliegen, der mit den bisher bekannten Einsatzmöglichkeiten für Adamantan-Derivate nicht im Einklang steht. Bekanntermaßen sind an den äußeren Haarzellen beispielsweise keine NMDA-Rezeptoren vorhanden, so daß eine antagonistische Wirkung für solche Rezeptoren ausscheidet. Auch eine Glutamat-Otoneurotoxizität wie sie von Ehrenberger/Felix postuliert wird, kann nicht in Frage kommen, da Glutamat für Haarzellen nicht toxisch ist.

Eine mögliche Erklärung kann darin liegen, daß die Adamantan-Derivate direkt auf andere, an den äußeren Haarzellen vorhandene Rezeptoren, beispielsweise Purinrezeptoren und Acetylcholinrezeptoren einwirken. Daneben gibt es Hinweise auf einen weiteren Mechanismus, der die Wirkung der Adamantan-Derivate mit nachfolgenden Schritten der Reizübermittlung in Zusammenhang bringt. Danach können die Adamantan-Derivate den Kationentransporter, der den Rücktransport von Neurotransmittern aus dem synaptischen Spalt in die Präsynapse vermittelt, hemmen. Dies führt zu einer Verarmung des Neurotransmitters in der efferenten Präsynapse und damit (indirekt) zu einer reduzierten Stimulation von Rezeptoren. Dieser an der Präsynapse, d. h. vor dem synaptischen Spalt, ansetzende Mechanismus könnte in der beschriebenen Form entlang der gesamten Gehörbahn bis zur Hörrinde wirken.

Bei der Erfindung kommen insbesondere die Tinnitusformen des chronischen Tinnitus, des subakuten Tinnitus oder auch des akuten Dauertinnitus für eine Behandlung in Frage, da gerade dieser Patientenkreis eine intensive Therapie benötigt.

Neben anderen Innenohrerkrankungen sind auch diejenigen, bei denen der Tinnitus auftritt, häufig mit einem Hörverlust (Schwerhörigkeit) verbunden. Beim Auftreten eines Tinnitus liegt dieser Hörverlust im Frequenzbereich des Tinnitus.

Die Adamantan-Derivate der Formel I können wie durch die Formel beschrieben oder vorzugsweise in Form ihrer pharmazeutisch akzeptablen Salze eingesetzt werden. Zu diesen Additionssalzen zählen beispielsweise die Hydrobromide, Sulfate, Acetate, Succinate, Tartrate oder insbesondere die Hydrochloride. In gleicher Weise können übliche andere Salze dargestellt und eingesetzt werden.

Die Menge der verwendeten Adamantan-Derivate ist normalerweise nicht kritisch und ergibt sich für den Fachmann in üblicher Weise durch Erfahrungswerte und/oder aus der Durchführung geeigneter Vorversuche vor dem Einsatz. Zweckmäßigerweise betragen die verwendeten Mengen üblicherweise zwischen etwa 0,01 bis etwa 100 mg/kg Körpergewicht, vorzugsweise von etwa 0,1 bis etwa 1 mg/kg Körpergewicht. Innerhalb des letztgenannten Bereichs sind Mengen von etwa 0,1 bis etwa 0,5 mg/kg Körpergewicht bevorzugt.

Wie erwähnt umfaßt die Erfindung die erfindungsgemäße Verwendung sämtlicher unter die Formel I fallender Aminoadamantane. Beispiele derartiger Verbindungen sind beispielsweise in der EP-B1-392059 aufgezählt.

Bevorzugte Verbindungen der Formel I sind diejenigen, bei denen R₁ und R₂ Wasserstoff H bedeuten sowie Verbindungen, bei denen R₅ und insbesondere zusätzlich R₁ und R₂ der Wasserstoffrest sind.

Bei den bereits genannten bevorzugten Verbindungen und auch bei anderen Verbindungen der Formel I sind die Reste R₃ und R₄ wahlweise ein Methyl- oder Ethylrest.

Eine besonders bevorzugte Verbindung, die nach der Erfindung einsetzbar ist, ist Memantin, d. h. 1-Amino-3,5-dimethyladamantan oder dessen Hydrochlorid Memantin-HCl. Diese Verbindung bzw. ihr Salz ist zur Behandlung des beschriebenen Tinnitus in besonderem Maße geeignet.

Eine bevorzugte Ausführungsform der erfindungsgemäßen Verwendung ergibt sich aus der nachfolgenden Beschreibung eines klinischen Anwendungsbeispiels. Dabei können die daraus hervorgehenden einzelnen Merkmale für sich allein oder in Kombination miteinander verwirklicht sein.

### Beispiel

Bei einer prospektiven klinischen Fallkontrollstudie wurden 104 Patienten mit einem das Adamantan-Derivat 1-Amino-3,5-dimethyl-adamantan (Memantin) enthaltenden Arzneimittel behandelt.

Alle Patienten litten an einem cochleären Tinnitus, der mit einer Schwerhörigkeit in dem Frequenzbereich verbunden war, in dem der Tinnitus auftrat. Ein positives Recruitment und eine deutliche Amplitudenreduktion bzw. Ausfall der otoakustischen Emissionen (OAE) lag vor, die mit üblichen audiometrischen Methoden bestimmt wurden. Die behandelte Gruppe von insgesamt 104 Patienten umfaßte Patienten mit chronischem Tinnitus, mit subakutem Tinnitus und mit akutem Dauertinnitus.

Als Medikament wurde das Präparat Akatinol Memantine® der Firma Merz & Co., GmbH & Co., Frankfurt am Main verwendet. Dieses Präparat enthält als Wirkstoff Memantin-HCl sowie als übliche pharmazeutische Träger und Hilfsmittel Lactose, Magnesiumstearat, Polyaminoethacrylat u. a. Derartige pharmazeutische Träger und Hilfsmittel können selbstverständlich in üblicher Weise bei sämtlichen Ausführungsformen der Erfindung vorhanden sein, so daß diese beispielsweise in Form von Tabletten, Dragees, sterilen Lösungen und Injektionen verabreichbar sind.

In der klinischen Studie wurde den Patienten der Wirkstoff zunächst durch Infusionen verabreicht. Bis zum 5. Tag erhielten sie üblicherweise eine Menge von 10 mg/d (die), ab dem 6. Tag wurde diese Menge auf 20 mg/d gesteigert. Je nach Patient trat bei der Personengruppe, die auf die Behandlung ansprach, zwischen dem 6. und dem 8. Tag eine schlagartige Besserung der Beschwerden auf.

Die Infusionsbehandlung wurde bis zum 10. Tag in einer Menge von 20 mg/d fortgesetzt. Ab dem 10. Tag wurden zwei Tabletten, die jeweils 10 mg des Wirkstoffs Memantin-HCl enthielten, verabreicht.

In einigen Fällen wurde höher dosiert, es wurde jedoch eine Menge von maximal 30 mg/d nicht überschritten.

Bei der Studie ergab sich, daß bei ca. 72 % der Patienten mit chronischem Tinnitus, bei 82 % mit subakutem Tinnitus und bei allen Patienten mit akutem Dauertinnitus eine dauerhafte Kompensation des Tinnitus erreicht wurde. Obwohl in den meisten Fällen zur Aufrechterhaltung der Wirkung kontinuierlich pro Tag zwei 10 mg-Tabletten verabreicht werden mußten, blieben einige Patienten auch nach Absetzen des Wirkstoffs beschwerdefrei.

Die Studie zeigt jedoch bereits jetzt deutlich eine wesentliche Verbesserung gegenüber bekannten Therapieversuchen, die in der Regel auch bei Dauermedikation nur eine vorübergehende Linderung der Beschwerden zeigten. In diesem Zusammenhang zeigen sich die Vorteile der Erfindung direkt im Vergleich mit einer Kontrollgruppe von Patienten, die mit dem bereits erwähnten Präparat Lidocain behandelt wurden. Auch diese Patientengruppe zeigte die beschriebene Innenohrfunktionsstörung mit chronischem Tinnitus, subakutem Tinnitus oder akutem Dauertinnitus. So war bei der Verabreichung von Lidocain der Anteil von Patienten, bei dem zwischen dem sechsten und zehnten Tag eine deutliche Besserung der Beschwerden auftrat, signifikant geringer.

## Patentansprüche

1. Verwendung von Adamantan-Derivaten der Formel 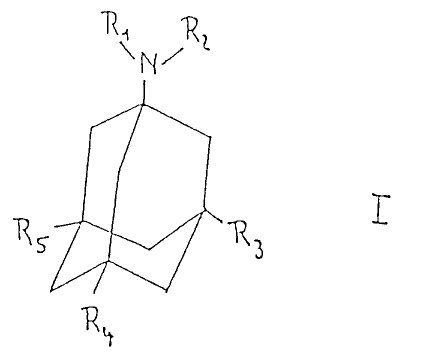 worin R₁ und R₂
- gleich oder verschieden sind und Wasserstoff oder geradkettige oder verzweigte Alkylgruppen mit 1 bis 6 C-Atomen sein können, oder
- zusammen mit dem N-Atom eine heterocyclische Gruppe mit 5 oder 6 Ringatomen darstellen können,
worin R₃ und R₄ gleich oder verschieden sind und Wasserstoff, geradkettige oder verzweigte Alkylgruppen mit 1 bis 6 C-Atomen, Cycloalkylgruppen mit 5 oder 6 C-Atomen oder die Phenylgruppe sein können,
und worin R₅ Wasserstoff oder eine geradkettige oder verzweigte Alkylgruppe mit 1 bis 6 C-Atomen sein können,
zur Herstellung eines Medikaments zur Behandlung von Tinnitus, der mit einem sogenannten positiven Recruitment und/oder einer Reduktion oder einem Ausfall der otoakustischen Emissionen verbunden ist.

2. Verwendung nach Anspruch 1, dadurch gekennzeichnet, daß es sich bei dem Tinnitus um einen subakuten oder chronischen Tinnitus handelt.

3. Verwendung nach Anspruch 1 oder Anspruch 2, dadurch gekennzeichnet, daß der Tinnitus mit einer Einschränkung des Hörvermögens, insbesondere im Frequenzbereich des Tinnitus, verbunden ist.

4. Verwendung nach einem der vorhergehenden Ansprüche, dadurch gekennzeichnet, daß die Adamantan-Derivate in Form ihrer pharmazeutisch akzeptablen Salze eingesetzt werden.

5. Verwendung nach einem der vorhergehenden Ansprüche, dadurch gekennzeichnet, daß die Adamantan-Derivate in einer Menge von ungefähr 0,01 bis 100 mg/kg Körpergewicht, vorzugsweise von ungefähr 0,1 bis 1 mg/kg Körpergewicht vorgesehen sind.

6. Verwendung nach Anspruch 5, dadurch gekennzeichnet, daß die Adamantan-Derivate in einer Menge von ungefähr 0,1 bis 0,5 mg/kg Körpergewicht vorgesehen sind.

7. Verwendung nach einem der vorhergehenden Ansprüche, dadurch gekennzeichnet, daß R₁ und R₂ Wasserstoffreste H sind.

8. Verwendung nach einem der vorhergehenden Ansprüche, dadurch gekennzeichnet, daß R₅ der Wasserstoffrest H ist.

9. Verwendung nach einem der vorhergehenden Ansprüche, dadurch gekennzeichnet, daß R₃ und R₄ Methyl- oder Ethylreste sind.

10. Verwendung nach einem der vorhergehenden Ansprüche, dadurch gekennzeichnet, daß das Adamantan-Derivat 3,5-Dimethyl-1-adamantanamin oder dessen Hydrochlorid ist.

## Claims

1. The use of adamantane derivatives of the formula 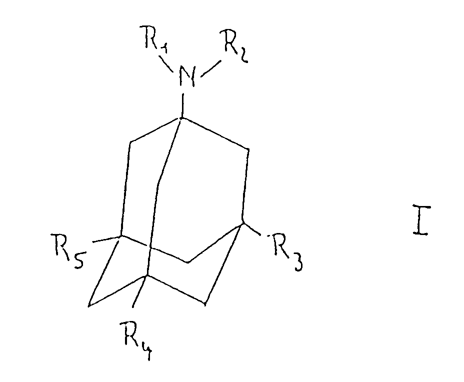 where R₁ and R₂
- are the same or different, and include hydrogen or straight or branched chain alkyl groups having 1 to 6 C atoms, or
- together with the N atom present a heterocyclic group having 5 or 6 ring atoms,
where R₃ and R₄ are the same or different, and include hydrogen, straight, or branched chain alkyl groups having 1 to 6 C atoms, cycloalkyl groups having 5 to 6 C atoms, or phenyl, and
where R₅ is hydrogen or a straight or branched chain alkyl group having 1 to 6 C atoms,
in the preparation of a medicament for the treatment of tinnitus which is associated with a socalled positive recruitment and/or a reduction or a failure of otoacoustic emissions.

2. The use according to claim 1, wherein the tinnitus is a subacute or a chronical tinnitus.

3. The use according to any of claim 1 or claim 2, wherein the tinnitus is associated with a reduction of audition, in particular in the range of frequency of the tinnitus.

4. The use according to any of the preceding claims, wherein the adamantane derivatives are applied in the form of pharmaceutically acceptable salts.

5. The use according to any of the preceding claims, wherein the adamantane derivatives are provided in an amount of about 0.01 to 100 mg/kg body weight, preferably of about 0.1 to 1 mg/kg body weight.

6. The use according to claim 5, wherein the adamantane derivatives are provided in an amount of about 0.1 to 0.5 mg/kg body weight.

7. The use according to any of the preceding claims, wherein R₁ and R₂ are hydrogen residues H.

8. The use according to any of the preceding claims, wherein R₅ is the hydrogen residue H.

9. The use according to any of the preceding claims, wherein R₃ and R₄ are methyl or ethyl residues.

10. The use according to any of the preceding claims, wherein the adamantane derivative is 3,5-dimethyl-1-adamantane amine or its hydrochloride.

## Revendications

1. Utilisation de dérivés de l'adamantane de formule 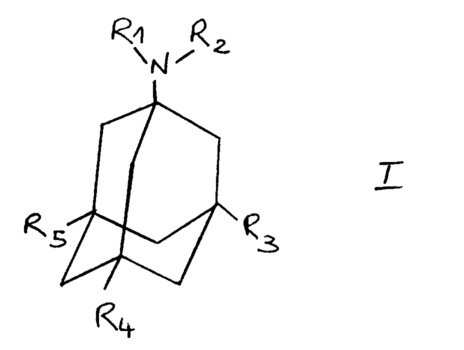 dans laquelle R1 et R2
- sont identiques ou différents et peuvent représenter chacun un atome d'hydrogène ou des groupes alkyles linéaires ou ramifiés ayant 1 à 6 atomes de carbone, ou
- qui, avec l'atome de N, peuvent représenter un groupe hétérocyclique ayant 5 ou 6 atomes ou chaînons de cycle,
et dans laquelle,
R3 et R4 sont identiques ou différents et peuvent représenter chacun un atome d'hydrogène, des groupes alkyles linéaires ou ramifiés comportant 1 à 6 atomes, des groupes cycloalkyles ayant 5 ou 6 atomes de carbone ou le groupe phényle,
et où R5 peut représenter un atome d'hydrogène ou un groupe alkyle linéaire ou ramifié ayant 1 à 6 atomes de carbone, pour la préparation d'un médicament destiné à traiter le tintement ou bourdonnement d'oreilles (Tinnitus) qui est lié à ce que l'on appelle un pronostic positif de récupération auditive et/ou une diminution ou une perte d'émissions otoacoustiques.

2. Utilisation selon la revendication 1, caractérisée en ce qu'il s'agit, pour le tintement ou bourdonnement d'oreilles (Tinnitus), d'un tintement subaigu ou chronique.

3. Utilisation selon la revendication 1 ou la revendication 2, caractérisée en ce que le tintement ou bourdonnement d'oreilles (Tinnitus) est lié à une limitation de la capacité auditive, en particulier dans le domaine des fréquences du tintement ou bourdonnement (Tinnitus) d'oreilles.

4. Utilisation selon l'une des revendications précédentes, caractérisée en ce que les dérivés de l'adamantane sont utilisés sous forme de leurs sels pharmaceutiquement acceptables.

5. Utilisation selon l'une des revendications précédentes, caractérisée en ce que les dérivés de l'adamantane sont prévus en une quantité d'environ 0,01 à 100 mg/kg de poids corporel, avantageusement environ 0,1 à 1 mg/kg de poids corporel.

6. Utilisation selon la revendication 5, caractérisée en ce que les dérivés de l'adamantane sont prévus en une quantité d'environ 0,1 à 0,5 mg/kg de poids corporel.

7. Utilisation selon l'une des revendications précédents, caractérisée en ce que R1 et R2 représentent des atomes d'hydrogène H.

8. Utilisation selon l'une des revendications précédentes, caractérisée en ce que R5 représente l'atome d'hydrogène H.

9. Utilisation selon l'une des revendications précédentes, caractérisée en ce que R3 et R4 représentent des groupements méthyle ou éthyle.

10. Utilisation selon l'une des revendications précédentes, caractérisée en ce que le dérivé de l'adamantane est la 3,5-diméthyl-1-adamantanamine ou son chlorhydrate.
